(19) Europäisches Patentamt
European Patent Office
Office européen des brevets

(11) Publication number: 0 061 231 A1

(12) # EUROPEAN PATENT APPLICATION

(21) Application number: 82300408.0

(22) Date of filing: 27.01.82

(51) Int. Cl.³: C 07 D 487/04
A 61 K 31/40
//(C07D487/04, 205/00, 209/00)

(30) Priority: 04.02.81 GB 8103349

(43) Date of publication of application: 29.09.82 Bulletin 82/39

(84) Designated Contracting States: BE CH DE FR GB IT LI LU NL SE

(71) Applicant: BEECHAM GROUP PLC
Beecham House Great West Road
Brentford Middlesex(GB)

(72) Inventor: Coulton, Steven
6 Badgers Close
Horsham W. Sussex(GB)

(72) Inventor: Corbett, David Francis
72E Holmesdale Road
Reigate Surrey(GB)

(72) Inventor: Eglinton, Alfred John
35 Smith Road
Reigate Surrey(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **Process for the preparation of azabicyclo(3.2.0)-hept-2-ene derivatives.**

(57) A process for the preparation of an antibacterially active compound of the formula (I):

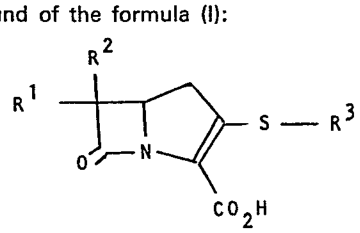

(I)

and salts and esters thereof wherein $R^1$ and $R^2$ independently represent hydrogen or an organic group, and $R^3$ is an optionally substituted aromatic or heteroaromatic moiety, which comprises reacting a compound of the formula (II):

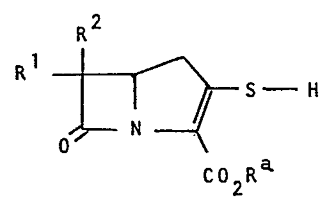

(II)

wherein $R^a$ is hydrogen or a carboxy-blocking group, with $X-R^3$ wherein X is a displaceable group. In addition certain compounds within the formula (II) are novel.



Croydon Printing Company Ltd.

CHEMICAL PROCESS

This invention relates to a chemical process for preparing carbapenem derivatives and in particular to 3-substituted thio carbapenem derivatives. These carbapenems are useful in the treatment of antibacterial infection either alone or in combination with other therapeutic agents.

European Patent Application Publication Nos. 0001627, 0001628, 0008514, 017970 and 017992 disclose a wide variety of carbapenem derivatives. We have found a new process for the preparation of certain of these compounds.

Accordingly the present invention provides a process for the preparation of a compound of the formula (I):

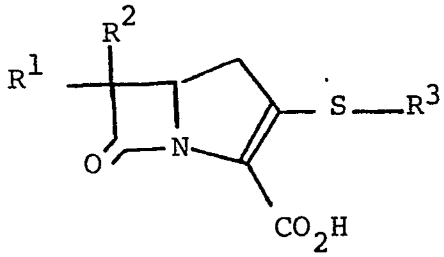

(I)

and pharmaceutically acceptable salts and in-vivo hydrolysable esters thereof wherein $R^1$ and $R^2$ independently represent a hydrogen atom or an organic group, and $R^3$ representsan optionally substituted aromatic or heteroaromatic moiety; which process comprises the reaction of a compound of the formula (II):

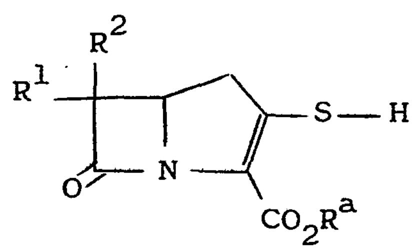

(II)

or reactive derivative thereof, wherein $R^1$ and $R^2$ are as defined in relation to formula (I) and $R^a$ is a carboxy-blocking group or a hydrogen atom; with a compound of the formula (III):

$$X \text{ —— } R^3 \qquad \text{(III)}$$

wherein X is a displaceable group, and thereafter if necessary:

i) removing any carboxy-blocking group $R^a$,

ii) converting the product into a pharmaceutically acceptable salt or in-vivo hydrolysable ester.

The process of this invention may be carried out in any solvent that is substantially inert during the reaction, for example tetrahydrofuran, dimethylformamide, dioxan, dimethoxyethane, dimethoxydiethyl-ether or hexamethylphosphoramide. Of these solvents dimethylformamide is preferred.

When the thiol compound of the formula (II) is used, the reaction is normally carried out in the presence of an acid acceptor, for example a carbonate or bicarbonate such as anhydrous potassium carbonate.

Alternatively we have found it useful to use a phase transfer catalyst. Particularly suitable phase transfer catalysts include tetrabutylammonium bromide and benzyldimethyl-n-hexadecyl ammonium bromide. Suitable solvents include halogenated water-immiscible solvents such as chloroform and dichloromethane in the presence of aqueous base such as aqueous sodium hydroxide.

The reaction is normally performed at a non-extreme temperature, for example $-30^{o}C$ to $+60^{o}C$, more suitably $-10^{o}C$ to $+40^{o}C$ and preferably at ambient temperature.

The reaction may employ either a compound of the formula (II) or a reactive derivative thereof.

Suitably X is a halo moiety such as bromo or chloro or is a sulphonate ester moiety such as a tosylate or mesylate. Of these preferably X is a chloro or bromo moiety.

Instead of using an acid acceptor in the reaction, a reactive derivative of the thiol may be used, preferably the reactive derivative is a salt of the thiol, in particular an alkali metal salt such as sodium or potassium.

The amount of compound of the formula (III) is generally between 1.0 and 2.0 moles per mole equivalent of the thiol of the formula (II) or reactive derivative thereof.

The group $R^3$ in the compound of the formulae (I) and (III) may be an optionally substituted aromatic or heteroaromatic moiety. Suitable substituents include those which are electron-withdrawing.

Examples of suitable electron-withdrawing substituents include nitro, cyano, chloro, fluoro, iodo, bromo, esterified sulphonyloxy or esterified carboxy wherein the ester group includes those of formula (a) - (f) as hereinafter defined, alkanoyl, arylcarbonyl, $C_{1-6}$ alkoxy, aryloxy, aralkoxy, sulphonamido, trifluoromethyl and and carbamoyl.

The group $R^3$ optionally may have further substituents either alone or in addition to one or more electron-withdrawing substituents, provided that they are not sufficiently electron-attracting so that they deactivate the aromatic or heteroaromatic nucleus and prevent reaction from occurring. For example further suitable substituents include $C_{1-6}$ alkyl, $C_{2-6}$ alkenyl, aryl such as phenyl, aryl $(C_{1-6})$ alkyl, aryl $(C_{2-6})$ alkenyl, heteroaryl, heteroaryl $(C_{1-6})$ alkyl, heterocyclyl, heterocyclyl $(C_{1-6})$ alkyl, $C_{1-6}$ alkylthio, arylthio such as phenylthio, hydroxy, mercapto, amino and protected amino such as $C_{1-6}$ alkylamino, di-$C_{1-6}$-alkylamino and $C_{1-6}$ alkanoylamino: wherein the hetero atom or atoms in the above-named heterocyclic and heteroaryl moieties are selected from one to four oxygen, sulphur or nitrogen atoms and the ring contains not greater than ten ring atoms.

Preferred substituents include $C_{1-6}$ alkyl such as methyl and ethyl, nitro, $C_{1-6}$ alkoxy such as methoxy and ethoxy, chloro, bromo, iodo and cyano.

An example of $R^3$ being an aromatic moiety is phenyl. Suitably when $R^3$ is a heteroaromatic moiety it is a 5 to 7 membered ring, preferably a 5- or 6-membered ring wherein the hetero atom or hetero atoms are selected from 1 to 4 oxygen, nitrogen or sulphut atoms. In one suitable aspect such a heteroaromatic moiety may be fused to another ring, for example a phenyl ring, so as to form a bicyclic aromatic ring system. Examples of suitable heteroaromatic ring systems include pyrimidinyl, triazinyl, thiazolyl, pyridyl, thiadiazolyl, oxazolyl, imidazolyl, benzimidazoyl, furyl and benzothiazolyl.

Preferably $R^3$ is an optionally substituted phenyl, pyrimidine or triazine group. In a further aspect $R^3$ is favourably an optionally substituted thiazolyl or pyridine group.

Preferably in the compounds of the formulae (I) and (II) either $R^1$ or $R^2$ is a hydrogen atom. In an alternative aspect both $R^1$ and $R^2$ are hydrogen atoms.

When the group $R^1$ or $R^2$ represents an organic radical, it may be substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the linear chain has 1-6 carbon atoms; heteroaralkyl, heterocyclyl and heterocyclylalkyl wherein the substituent or substituents relative to the above-named radicals are selected from the group consisting of: amino, mono-, di- and tri-alkylamino, hydroxyl, alkoxyl, mercapto, alkylthio, arylthio such as phenylthio, sulfamoyl, amidino, guanidino, nitro, chloro, bromo, fluoro, cyano and carboxy, and wherein the hetero atom or atoms in the above-named heterocyclic moieties are selected from the group consisting of 1-4 oxygen, nitrogen or sulphur atoms, and wherein the alkyl moieties of the above-recited substituents have 1-6 carbon atoms.

Preferably one of $R^1$ and $R^2$ is a hydrogen atom and the other is sulphonato-oxyethyl or hydroxyalkyl containing up to 6 carbon atoms, in particular α-hydroxyethyl or α-hydroxypropyl. In a favoured aspect one of $R^1$ and $R^2$ is hydrogen and the other is sulphonato-oxyethyl or α-hydroxyethyl.

In the compounds of the formulae (I) and (II) if $R^1$ is a hydrogen atom and $R^2$ is not a hydrogen atom, or vice versa, then said compounds may have the cis- or trans-configuration about the β-lactam ring, that is to say they have the (5R,6R) or 5R,6S) configuration. Alternatively the compounds of the formulae (I) and (II) may be presented in the form of a cis/trans mixture.

Suitable carboxyl-blocking derivatives for the group $-CO_2R^a$ in formula (II) include salts, esters, and anhydride derivatives of the carboxylic acid. The derivative is one which may readily be cleaved at a later stage of the reaction. The salts need not be pharmaceutically acceptable. Suitable salts include inorganic salts, for example metal salts such as silver or mercuric salt, or alkali metal salts such as the lithium or sodium salt, tertiary amine salts, such as those with tri-lower-alkylamines, N-ethylpiperidine, dimethylpiperazine. A preferred salt is with triethylamine.

Suitable ester-forming carboxyl-blocking groups are those which may be removed under conventional conditions. Such groups for $R^a$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, an oxime radical of formula $-N{=}CHR^o$ where $R^o$ is aryl or heterocyclic, or an *in vivo* hydrolysable ester.

The carboxyl group may be regenerated from any of the above esters by usual methods appropriate to the particular $R^a$ group, for example, acid - and base - catalysed hydrolysis, or by enzymically - catalysed hydrolysis, or by hydrogenation. The hydrolysis must of course be carried out under conditions to which the groups on the rest of the molecule are stable.

When it is desired to produce a compound of formula (I) in the form of a free acid or salt by the process of this invention, a compound of formula (II) is generally employed wherein $R^a$ is a carboxyl-blocking group. For the preparation of a compound of formula (I) in the form of a pharmaceutically acceptable ester, it is convenient to employ a compound of formula (II) wherein $R^a$ represents the desired ester group.

Preferably the process of this invention is performed on a compound of the formula (II) wherein $R^a$ is an ester-forming group.

Suitable esters of the compounds of the formula (II) for use in the process of this invention include those cleavable by biological methods and by chemical methods such as hydrogenolysis, hydrolysis, electrolysis or photolysis.

Suitably the carboxylic acid is esterified by a group of the sub-formula (a), (b), (c), (d), (e) or (f):

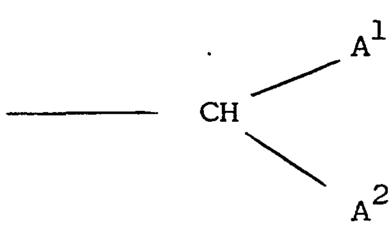

(a)

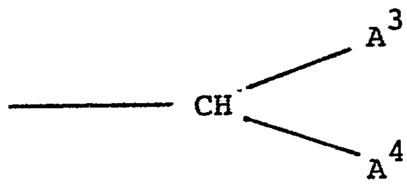

(b)

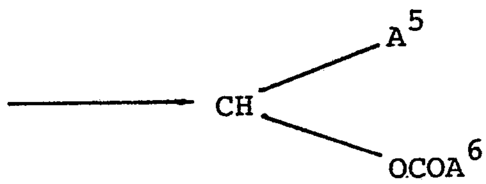

(c)

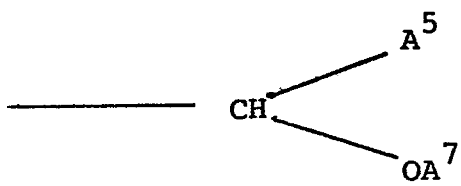

(d)

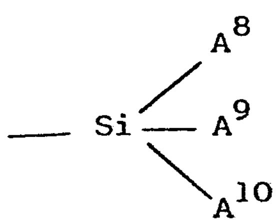

(e)

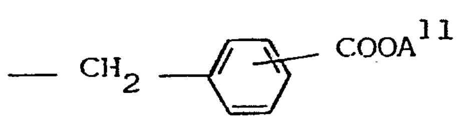

(f)

wherein $A^1$ is a hydrogen atom, $C_{1-6}$ alkanoyl or an alkyl, alkenyl or alkynyl group of up to 3 carbon atoms; $A^2$ is a hydrogen atom or a methyl group; $A^3$ is a phenyl group or a phenyl group substituted by a fluorine, chlorine or bromine atom or a nitro, methyl or methoxy group; $A^4$ is a hydrogen atom or a phenyl group or a phenyl group substituted by a fluorine, chlorine or bromine atom or a nitro, methyl or methoxy group; $A^5$ is a hydrogen atom or a methyl group; $A^6$ is a $C_{1-4}$ alkyl, phenyl or $C_{1-4}$ alkoxy group or $A^5$ is joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacyl or bromophenacyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically accptable in-vivo hydrolysable esters are those esters which hydrolyse in the human body to produce the parent acid or its salt. Such esters may be identified by administration to a test animal such as a rat or mouse by intravenous administration and thereafter examining the test animal's body fluids for the presence of the compound of the formula (I) or its salt.

Suitable esters of this type include those of sub-formula (c) as hereinbefore defined.

Suitable pharmaceutically acceptable salts include those of the alkali and alkaline earth metals, of these the sodium and potassium salts are preferred. These pharmaceutically acceptable salts may be formed at the C-2 carboxy

and/or at the C-6 sulphonato-oxyethyl moiety (if present). Thus compounds of the formula (I) wherein $R^1$ contains a $OSO_3H$ group or pharmaceutically acceptable salt thereof, may be in the form of a di-salt such as the di-sodium or dipotassium salt, or may be in the form of a mono-salt of an in-vivo hydrolysable ester, or may be in the form of a mono-salt of an acid or may be in the form of a di-acid.

Non-pharmaceutically acceptable salts of compounds of the formula (I) are also of use as they may be converted to pharmaceutically acceptable salts or in-vivo hydrolysable esters. An example of such a salt is the lithium salt.

Esters may be converted to the free acid or a salt of the formula (I) by methods appropriate to the particular ester, for example by acid - and base - catalysed hydrolysis or by enzymically-catalysed hydrolysis or via hydrogenolysis, electrolysis or photolysis. The conditions selected must of course not cause substantial degradation of the rest of the molecule.

Compounds of the formula (I) when in the form of an in-vivo ester, may be prepared from the free acid or salt thereof by esterification using methods known to those skilled in the art. A convenient method is often reaction with a reactive halide, for example bromophthalide in solution in a polar organic solvent such as dimethylformamide.

The compounds of the formula (II) may be prepared by the methods of European Patent Application Publication Nos. 0001627 and 0001628 and European Patent Application No. 80302659.0. (Publication No. 0024832).

In another aspect of this invention we provide a compound of the formula (IV) or a pharmaceutically acceptable salt or in-vivo hydrolysable ester thereof:

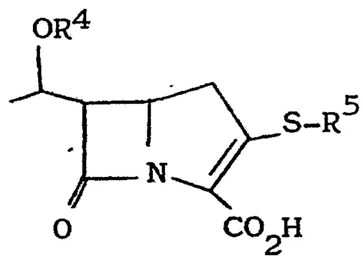

(IV)

wherein $R^4$ is hydrogen or a $SO_3H$ moiety, and $R^5$ is an optionally substituted triazinyl group. These compounds are a novel class of antibiotics not specifically disclosed in any of European Patent Application Publication Nos. 0001627, 0001628, 0008514, 0017970 and 0017992.

In a further aspect of this invention, therefore there is provided a pharmaceutical composition which comprises a compound of the formula (IV) or a pharmaceutically acceptable salt or in-vivo hydrolysable ester and a pharmaceutically acceptable carrier.

The compositions of this invention may be prepared by conventional methods of preparing antibiotic compositions and in conventional manner may be adapted for oral, topical or parenteral administration.

Aptly, the compositions of this invention are in the form of a unit-dose composition adapted for oral administration.

Alternatively the compositions of this invention are in the form of a unit-dose composition adapted for administration by injection.

Unit dose forms according to this invention will normally contain from 50 to 500mgs of a compound of this invention, for example about 62.5, 100, 125, 150, 200, 250 or 300mgs. Such compositions may be administered from 1 to 6 times a day or more conveniently 2, 3 or 4 times a day so that the total daily dose for a 70kg adult is about 200 to 2000mgs, for example about 400, 600, 750, 1000 or 1500mgs.

The compositions of this invention may be used to treat bacterial infection, in animals such as mammals including humans,

for example infections of the respiratory tract, urinary tract or soft tissues, or mastitis in cattle.

The carriers used in the compositions of this invention may include diluents, binders, disintegrants, lubricants, colours, flavouring agents or preservatives in conventional manner. Thus suitable agents include lactose, starch, sucrose, calcium phosphate, sorbitol, polyvinylpyrrolidone, acacia, gelatin, tragacanth, potato starch or polyvinylpolypyrrolidone, magnesium stearate or sodium lauryl sulphate.

Orally administrable forms of the compositions of this invention are most suitably in the form of unit-dose units such as tablets or capsules.

The following Examples illustrate the invention.

Example 1

p-Nitrobenzyl (5R,6S)-3-[(4-chloro-6-methyl-pyrimidin-2-yl)-thio]-6-[(S)-1-hydroxyethyl]-7-oxo-1-azabicyclo[3.2.0]-hept-2-ene-2-carboxylate

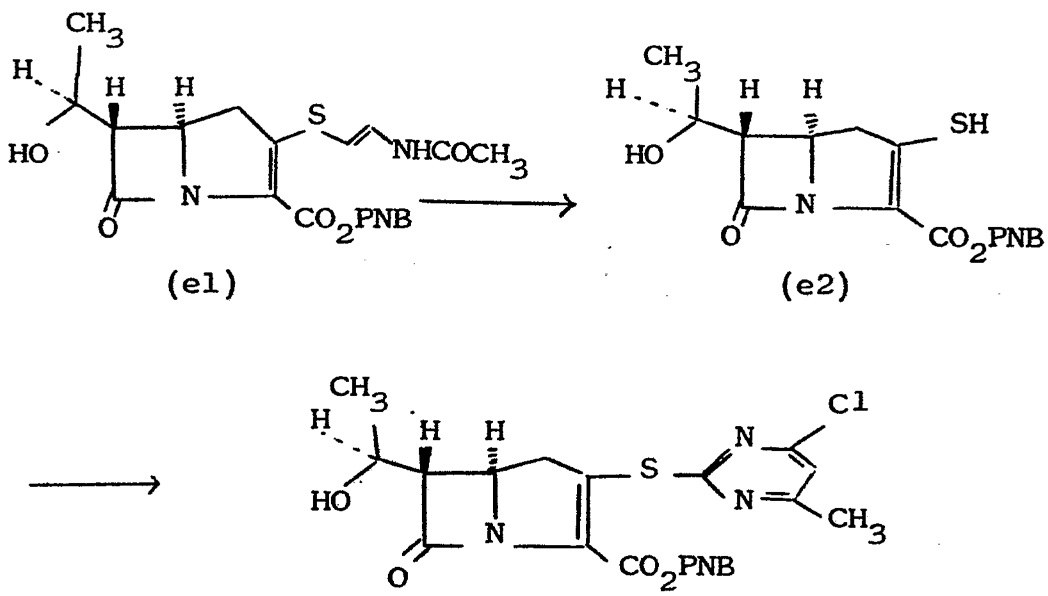

(e1) (e2) (e3)

p-Nitrobenzyl (5R,6S)-3-[E-2-acetamidoethenyl-thio]-6-[(S)-1-hydroxyethyl]-7-oxo-1-azabicyclo[3.2.0]-hept-2-ene-2-carboxylate (e1) (500 mg; 1.119 mm) was dissolved in 1,4-dioxan (10 ml) containing water (30 drops). A solution of N-bromoacetamide (155 mg, 1.119 mm) in 1,4-dioxan (5 ml) was then added and the solution stirred at room temperature for 4.5 minutes. Chloroform (50 ml) was added and the organic phase was washed with pH 7.0, 0.05M phosphate buffer, saturated sodium chloride solution and dried over anhydrous magnesium sulphate. Filtration and removal of the solvent at reduced pressure yielded the crude thiol (e2) as a gum, $\nu_{max}$ ($CHCl_3$) 1780, 1730, 1700 $cm^{-1}$.

The crude thiol was dissolved in dry dimethyl formamide (10 ml) and stirred at room temperature for 25 minutes with anhydrous potassium carbonate (155 mg) and 2,4-dichloro-6-methylpyrimidine (182 mg; 1.119 mm). Ethyl acetate was added and the organic solution washed with water, saturated sodium chloride solution and dried over anhydrous magnesium sulphate. After filtration, the solvent was removed at reduced pressure to yield the crude product, which was chromatographed over silica gel (40 gm). Elution with 2% ethanol/chloroform afforded the title compound (e3) as a white solid. This solid was triturated with ethyl acetate/diethyl ether and collected by filtration (102 mg) $\nu_{max}$ (KBr) 3400, 1802, 1714, 1610, 1565, 1512, 1350, 1332 $cm^{-1}$, $\lambda_{max}$ (EtOH) 326 nm (Em 14,530), 265 nm (Em 13,510), $\delta_H$ ($\delta_7$ - DMF) 8.27 (2H, d, aromatic protons), 7.82 (2H, d, aromatic protons), 7.61 (1H, S, pyrimidine proton), 5.52 (2H, q, $\underline{CH_2}Ar$), 5.23 (1H, d, O$\underline{H}$, exchanges with $D_2O$), 4.42 (1H, dt, 5-C$\underline{H}$), 4.15 (1H, broad res., 8-C$\underline{H}$), 3.1-3.8 (3H, t + $\underline{ABX}$, 6-C$\underline{H}$ + 4-$\underline{CH_2}$), 2.48 (3H, s, pyrimidine $\underline{CH_3}$), 1.31 (3H, d, C$\underline{H_3}$CH), m/s (relative intensity %) 490.0703 (2) ($C_{21}H_{19}N_4O_6SCl$ required 490.0710), 446(8), 407(38), 405(100), 361(10), 287(50), 286(40), 266(45), 160(45), 136(70), 107(60), 106(70).

## Example 2

**p-Nitrobenzyl (5R,6S)-3-[4,6-dimethoxy-1,3,5-triazin-2-ylthio]-6-(S)-1-hydroxyethyl]-7-oxo-1-azabicyclo[3.2.0] hept-2-ene-2-carboxylate**

(el) →

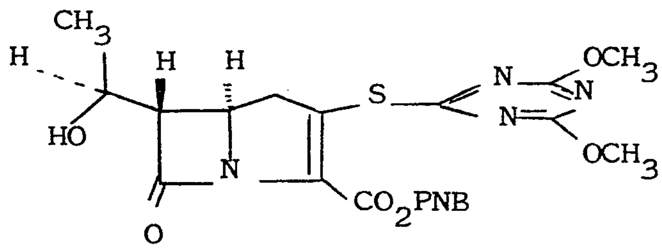

(e4)

The title compound (e4) (75 mg) was obtained as a white crystalline solid by the reaction of the thiol (e2), derived from the ester (el) (250 mg) with 2-chloro-4,6-dimethoxytriazine, by the method described in example 1., $\lambda_{max}$ (EtOH) 310 nm (Em 12441), 260 nm (Em 13436), $\nu_{max}$ (KBr) 3415 (broad), 1782, 1693, 1605 $cm^{-1}$, $d_H$ ($d_7$-DMF) 8.25 (2H, d, aromatic H), 7.81 (2H, d, aromatic H), 5.50 (2H, q, C<u>H</u>$_2$Ar), 5.22 (1H, d, O<u>H</u>), 4.0-4.5 (2H, m, 5-C<u>H</u> + 8-C<u>H</u>), 4.00 (6H, s, 2 x OC<u>H</u>$_3$), 3.2-3.9 (2H, ABX, 4-C<u>H</u>$_2$), 3.66 (1H, t, 6-C<u>H</u>), 1.31 (3H, d, C<u>H</u>$_3$CH), m/e (relative intensity %) 503.1135 ($M^+$, 20%, $C_{21}N_5O_8S$ requires 503.1110), 459 (5), 418 (60), 330 (20), 286 (70), 279 (30), 173 (100), 136 (45), 106 (55).

Example 3

p-Nitrobenzyl (5R,6R)-3-[4,6-dimethoxy-1,3,5-triazin-2-ylthio]-6-[(S)-1-hydroxyethyl]-7-oxo-1-azabicyclo[3.2.0]-hept-2-ene-2-carboxylate

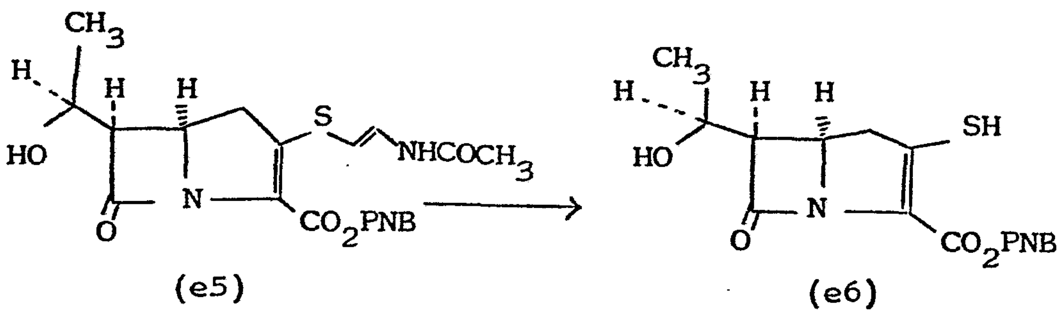

(e5) (e6)

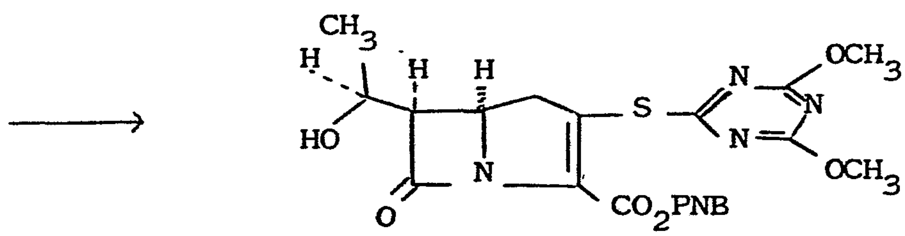

(e7)

The title compound (e7) (95 mg) was prepared as a white crystalline solid by reaction of the thiol (e6), derived from p-nitrobenzyl (5R,6R)-3-(E-2-acetamidoethenyl-thio)-6-[(S)-1-hydroxyethyl]-7-oxo-1-azabicyclo[3.2.0]hept-2-ene-2-carboxylate (e5) (250 mg) with 2-chloro-4,6-dimethoxy-triazine by the procedure described in example 1., $\lambda_{max}$ (EtOH) 310 nm (Em 12468) 260 nm (Em 13501), $\nu_{max}$ (KBr) 3400, 1780, 1700, 1607, 1530 (broad), 1350 $cm^{-1}$ (broad), $d_H$ ($d_7$-DMF) 8.27 (2H, d, aromatic protons), 7.82 (2H, d, aromatic protons), 5.49 (2H, q, $C\underline{H}_2Ar$), 5.09 (1H, d, O$\underline{H}$, exchanges with $D_2O$), 4.0-4.6 (2H, m, 5-CH + 8-C$\underline{H}$), 4.0 (6H, s, 2 x $OC\underline{H}_3$), 3.6-3.9 (3H, m, 4-$C\underline{H}_2$ + 6-C$\underline{H}$), 1.31 (3H, d, $C\underline{H}_3CH$), m/e (relative intensity %)

503.1086 ($M^+$, 4%, $C_{21}H_{21}N_5O_8S$ requires 503.1110), 459 (5), 418 (50), 368 (25), 330 (25), 286 (56), 279 (30), 173 (100).

Example 4

(5R,6R)-3-[4,6-dimethoxy-1,3,5-triazin-2-ylthio]-6-[(S)-1-hydroxyethyl]-7-oxo-1-azabicyclo[3.2.0]hept-2-ene-2-carboxylic acid

(e7) ⟶

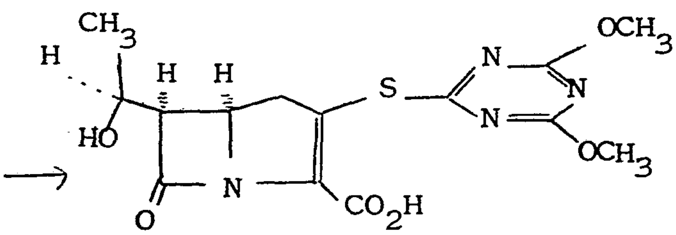

(e8)

A solution of the ester (e7) (40 mg) in 1,4-dioxan (10 ml), water (1.5 ml), ethanol (0.4 ml) and 0.05 M pH 7.0 phosphate buffer (2 ml) was hydrogenated in the presence of 5% palladium on carbon (60 mg) for 2 hours. The suspension was then filtered over Celite, washing well with water (25 ml). The filtrate was concentrated to approximately 20 ml and washed with ethyl acetate (3 x 25 ml). The resulting aqueous solution was estimated to contain 20 mg of the title compound (e8), based on Em 9,500 at $\lambda_{max}$ 291 nm in the U.V. spectrum.

Example 5

p-Nitrobenzyl (5R,6R)-3-[5-nitropyrimid-2-yl)thio]-6-[(S)-1-hydroxyethyl]-7-oxo-1-azabicyclo[3.2.0]hept-2-ene-2-carboxylate

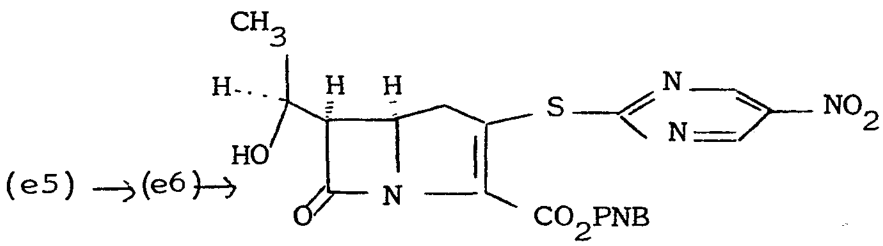


(e9)

p-Nitrobenzyl (5R,6R)-3-[E-2-acetamidoethenylthio]-6-[(S)-1-hydroxyethyl]-7-oxo-1-azabicyclo[3.2.0]hept-2-ene-2-carboxylate (e5) (250 mg; 0.56 mM) was dissolved in 1,4-dioxan (5 ml) containing water (15 drops). A solution of N-bromoacetamide (78.5 mg; 0.56 mM) in 1,4-dioxan (3 ml) was then added and the solution stirred at room temperature for 4.5 minutes. Chloroform (25 ml) was added and the organic phase was washed with pH 7.0, 0.05 M phosphate buffer, saturated sodium chloride solution and dried over anhydrous magnesium sulphate. Filtration and removal of the solvent at reduced pressure yielded the crude thiol (e6) as a gum. The thiol was then dissolved in chloroform (20 ml) and layered with water (15 ml). 2-Chloro-5-nitropyrimidine (180 mg; 1.119 mM), cetyl-dimethylbenzyl ammonium chloride (50 mg) and O.IN sodium hydroxide solution (5.6 ml; 0.56 mM) were added and the two phase system stirred vigorously at room temperature for 35 minutes.

The mixture was diluted with dichloromethane (100 ml) and water (100 ml) and the organic layer washed with saturated sodium chloride solution, and dried over anhydrous magnesium sulphate. Filtration and removal of the solvent at reduced pressure gave the crude product, which was chromatographed over silica gel (15 gm). Elution with chloroform yielded the title compound (e9) as a yellow foam. Crystallisation from ethyl acetate/ether gave a bright yellow solid (75 mg), $\lambda_{max}$ (EtOH) 340 nm (Em 13850), 267 nm (Em 15810), $\nu_{max}$ (KBr) 3510 (broad), 1776, 1709, 1603, 1578, 1560, 1512, 1341, 1322 $cm^{-1}$, $\delta_H$ ($CDCl_3$) 9.27 (2H, s, pyrimidine protons), 8.22 (2H, d, aromatic protons), 7.63 (2H, d, aromatic protons), 5.38 (2H, q, $C\underline{H}_2Ar$), 4.0-4.65 (2H, m, 5-$C\underline{H}$ + 8-$C\underline{H}$), 3.55-3.80 (3H, m, 4-$C\underline{H}_2$ + 6-$C\underline{H}$), 1.79 (1H, d, $O\underline{H}$), 1.44 (3H, d, $C\underline{H}_3CH$), m/e (relative intensity %) 443 (2), 401.0397 (5%, $C_{16}H_{11}N_5O_6S$ requires 401.0430), 330 (5), 286 (25), 263 (15), 221 (16), 157 (30), 136 (45), 106 (70), 78 (100).

Example 6

(5R,6R)-3-[(5-aminopyrimid-2-yl)thio]-6-[(S)-1-hydroxyethyl]-7-oxo-1-azabicyclo[3.2.0]hept-2-ene-2-carboxylic acid

(e9)

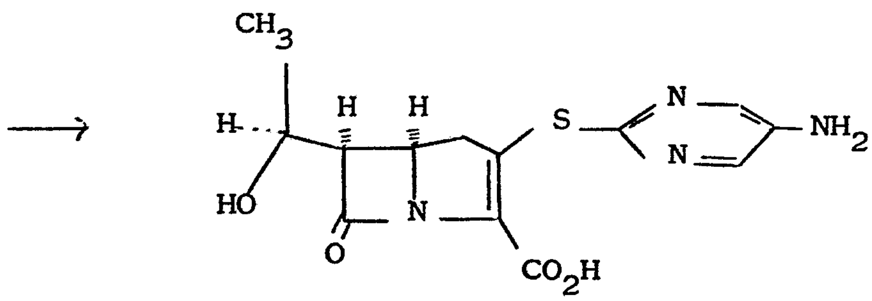

(e10)

An aqueous solution containing the title compound (e10) was obtained by hydrogenolysis of the ester e(9) (15 mg). The procedure outlined in example 4, was followed with the exceptions that the hydrogenolysis time was extended to 4 hours and 10% palladium on carbon catalyst was used. The solution was estimated to contain 10.7 mg of product based on Em 12,000 at $\lambda_{max}$ 305 nm in the u.v. spectrum, $\lambda_{max}$ ($H_2O$) 305 nm, 267 nm.

Example 7

Tetra-n-butylammonium salt of p-nitrobenzyl (5R,6R)-6-[(S)-1-hydroxysulphonyloxyethyl]-3-(5-nitropyrimid-2-ylthiol)-7-oxo-1-azabicyclo [3.2.0]hept-2-ene-2-carboxylate

Step A

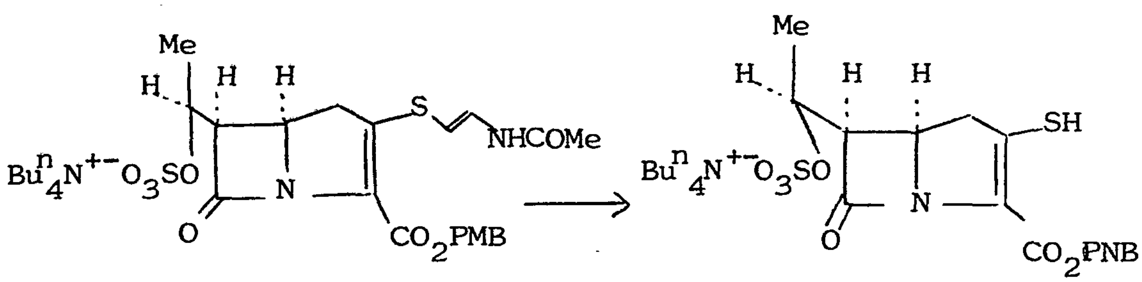

(e11) (e12)

The tetra-n-butylammonium salt (e11) (200 mg) in 15% aqueous dioxan was treated with N-bromoacetamide (36 mg) in dioxan. After stirring at room temperature the solution was diluted with chloroform, and the organic layer was washed with pH7 buffer solution and dilute brine. Evaporation of the dried ($MgSO_4$) organic layer yielded the thiol (e12) as a foam (156 mg); $\nu_{max}$ ($CHCl_3$) 1780 and 1705 $cm^{-1}$.

Step B

(e12) →

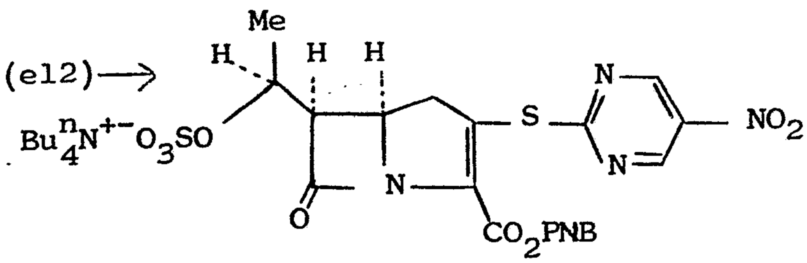

(e13)

A solution of the thiol (e12) and 5-nitro-2-chloropyrimidine (55 mg) in chloroform (5 ml) was stirred vigorously with an aqueous solution of sodium hydroxide (9 mg in 2.25 ml). After 40 min at room temperature, chloroform (30 ml) was added and the organic layer was washed with water (30 ml) and dried ($MgSO_4$). The solution was evaporated in vacuo and the residue was chromatographed on silica-gel using a gradient elution from chloroform to 20% ethanol in chloroform. Fractions containing the product were collected and evaporated in vacuo to afford the title compound (e13) as a gum (34 mg); $\lambda_{max}$ (EtOH) 336 and 267 nm; $\nu_{max}$ ($CHCl_3$) 1780 and 1725 $cm^{-1}$; $\delta$ ($CDCl_3$) 0.99 (12H, t, J Hz, 4 x $C\underline{H}_3CH_2CH_2$), 1.43 (8H, m, 4 x $CH_2C\underline{H}_2CH_3$), 1.63 (11H, m, 4 x $C\underline{H}_2CH_2CH_3$ and $C\underline{H}_3CH$), 3.25 (8H, m, 4 x $NC\underline{H}_2CH_2$), 3.52 (1H, dd, J 10.5 and 19.5 Hz, 4-CHa), 3.83 (1H, dd, J 5.5 and 11 Hz, 6-CH), 4.07 (1H, dd, J 19.5 and 9.5 Hz, 4-CHb), 4.51 (1H, dt, J, 5.5 5.5 and 9.5 Hz, 5-CH), 4.85 (1H, m, $C\underline{H}CH_3$), 5.28 and 5.50 (each 1H, d, J 14 Hz, $C\underline{H}_2C_6H_4NO_2$); 7.65 and 8.22 (each 2H, d, J 9 Hz, $C_6\underline{H}_4NO_2$) and 9.31 (2H, s, 2 x pyrimidyl-CH).

Example 8

Sodium (5R,6R)-3-(5-aminopyrimidyl-2-thio)-6-[(S)-1-hydroxysulphonyloxyethyl]-7-oxo-1-azabicyclo [3.2.0]hept-2-ene-carboxylate

(e13) ⟶ 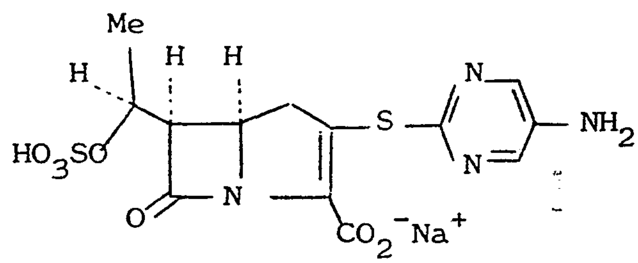

(e14)

A mixture of the mono-ester (e13) (180 mg), 10% Pd on C (180 mg), dioxan (15 ml) water (5 ml) and 0.05 M pH7 phosphate buffer solution (5 ml) was shaken under hydrogen for 3.5 h. To the mixture was added a solution of sodium tetrafluoroborate (24 mg) in water (1 ml), before filtering over Celite washing well with water (40 ml). The solution was concentrated in vacuo to a volume of Ca 35 ml and was then washed with ethyl acetate (3 x 50 ml). The aqueous layer was further concentrated to small volume and then chromatographed on a column of Biogel P2 (3.5 x 25 cm) eluting with water. Fractions containing chromophores at $\lambda_{max}$ ($H_2O$) 304 and 268 nm in the u.v. spectrum were combined to afford an aqueous solution of the title compound (e14). A portion of the solution was freeze-dried to afford the product as a solid.

Example 9

p-Nitrobenzyl(5R,6R)-3-[(5-nitrothiazol-2-yl)thio]-6-[(S)-1-hydroxyethyl]-7-oxo-1-azabicyclo[3.2.0]hept-2-ene-2-carboxylate

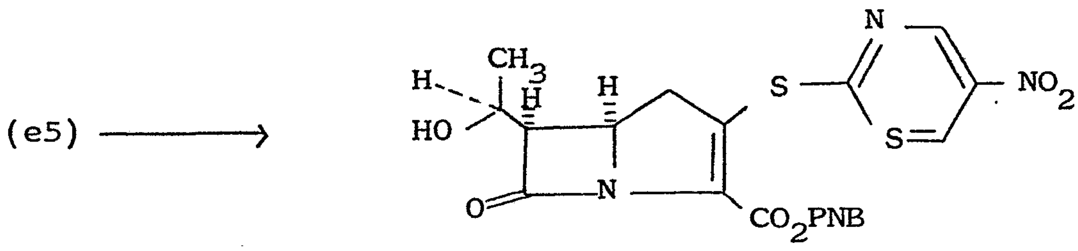

(e9)

Reaction of the thiol (e6) derived from the ester (e5) (200 mg) with 2-bromo-5-nitrothiazole, following the procedure outlined in Example 1, afforded the title compound (e9) as a yellow crystalline solid (35 mg), $\lambda_{max}$ (EtOH) 366 nm ($\epsilon$m 8718), 293 nm ($\epsilon$m 12,839), 266 nm ($\epsilon$m 13948), $\nu_{max}$ ($CH_2Cl_2$) 3590, 1779, 1700, 1602, 1522, 1330 $cm^{-1}$, $\delta_H$ ($CDCl_3$) 8.50 (1H, s, triazole-CH), 8.26 (2H, d, aromatic protons), 7.67 (2H, d, aromatic protons), 5.42 (2H, q, $CH_2Ar$), 4.41 (1H, dq, 5-CH), 4.23 (1H, m, 8-CH), 3.66 (1H, dd, $J_{56}$ 5.9Hz, $J_{68}$ 8.3Hz, 6-CH), 3.22-3.61 (2H, ABX, 4-$CH_2$, $J_{4a5}$ 8.9Hz, $J_{4b5}$ 10.0Hz, $J_{4a4b}$ 18.2Hz), 1.43 (3H, d, $CH_3CH$), m/e (relative intensity%) 330 (24), 286 (37), 259 (8), 194 (15), 162 (100), 152 (32), 136 (65), 106 (90), 78 (85), no $M^+$.

Example 10

p-Nitrobenzyl (5R,6R)-3-[(5R,6R)-3-[(5-nitropyrid-2-yl) thio]-6-[(S)-1-hydroxyethyl]-7-oxo-1-aza-bicyclo[3.2.0] - hept-2-ene-2-carboxylate

(e5) 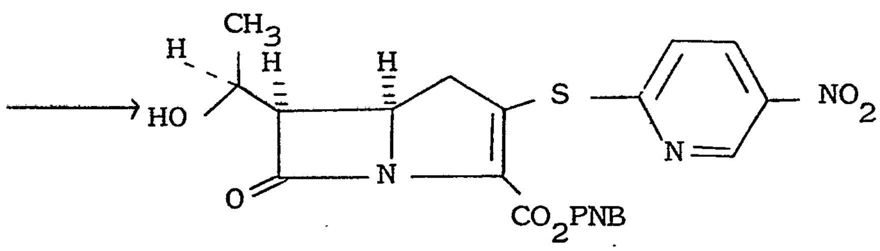

(e11)

Reaction of the thiol (e6) derived from the ester (e5) (250 mg) with 2-chloro-5-nitropyridine, following the procedure outlined in Example 1, gave the title compound (e11) as a yellow crystalline solid (72 mg), $\lambda_{max}$ (EtOH) 350 nm ($\varepsilon_m$ 14389), 265 nm ($\varepsilon_m$ 14717), $\nu_{max}$ (KBr) 3540, 1781, 1722, 1608, 1589, 1568, 1517, 1349, 1321 $cm^{-1}$, $\delta_H$ ($CDCl_3$) 9.29 (1H, d, pyridine-6-CH), 8.35 (1H, dd, pyridine-4-CH), 8.21 (2H, d, aromatic protons), 7.63 (2H, d, aromatic protons), 7.51 (1H, d, pyridine-3-CH), 5.38 (2H, q, $CH_2Ar$), 4.43 (1H, dq, $J_{56}$ 6Hz, 5-CH), 4.0-4.4 (1H, m, 8-CH), 3.63 (1H, dd, $J_{68}$ 8.5Hz, 6-CH), 3.48 (2H, d, $J_{45}$ 10Hz, 4-$CH_2$), 1.79 (1H, d, OH), 1.40 (3H, d, $CH_3CH$), m/e (relative intensity %) 401 (1), 330 (10), 286 (40), 156 (100), no $M^+$.

Example 11

Sodium salt of p-nitrobenzyl (5R, 6R)-6-[(S)-1-hydroxysulphonyloxyethyl]-3-(5-nitrothiazol-2-ylthio)-7-oxo-1-azabicyclo [3.2.0]hept-2-ene-2-carboxylate

Step A

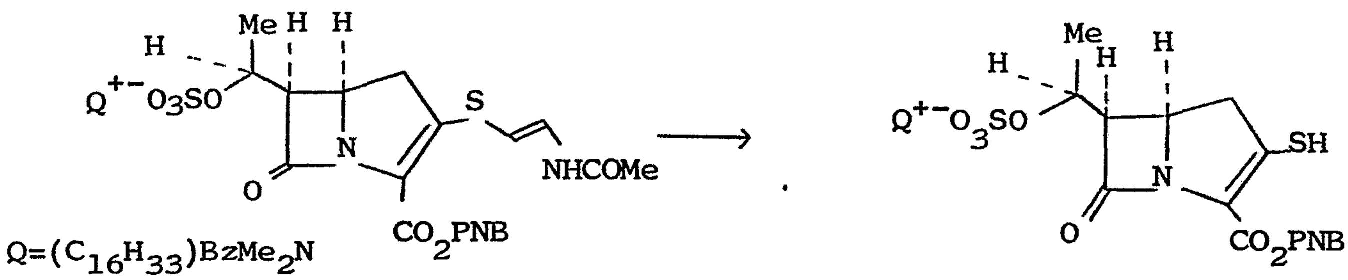

$Q=(C_{16}H_{33})BzMe_2N$

(e15) (e16)

The cetylbenzyldimethylammonium salt (e15) (1.47) was dissolved in 15% aqueous dioxan (23 ml) and to the solution was added a solution of N-bromoacetamide (229 mg) in dioxan (1 ml). After strring at room temperature for 4.5 min. the solution was diluted with chloroform (70 ml) and the organic layer was washed with pH 7 buffer solution (50 ml) and dilute brine (30 ml). Evaporation of the dried ($MgSO_4$) organic layer afford a foam which contained the thiol (e16).

Step B

(e16) →

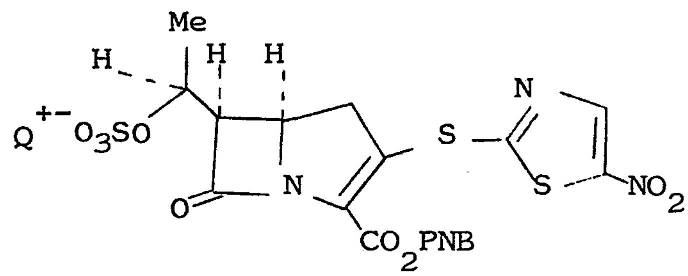

(e17)

The product from step A was dissolved in DMF (20 ml) and the solution was stirred with 5-nitro-2-bromothiazole (570 mg) and dry potassium carbonate (229 mg) for 25 min. Ethyl acetate (70 ml) was added and the solution was washed with dilute brine (3 x 70 ml). Evaporation of the dried ($MgSO_4$) solution gave a foam which was chromatographed on a column of silica gel using a gradient elution of chloroform to 20% ethanol-chloroform. The quaternary ammonium salt (e17) was obtained as a yellow foam (400 mg); $\lambda_{max}$ (EtOH) 365, 288sh and 267 nm $\nu_{max}$ ($CHCl_3$) 1780 1720sh and 1700cm$^{-1}$.

Step C

(e17) →

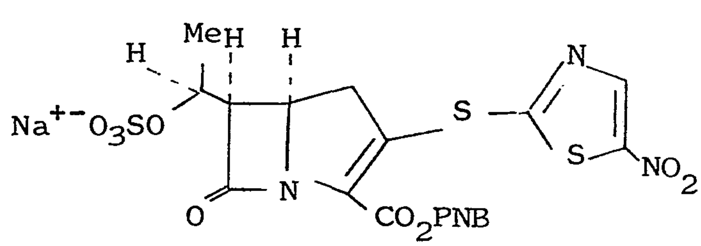

(e18)

The product from step B was dissolved in acetone (4 ml) and to this solution was added a solution of sodium iodide (80 mg) in acetone (1 ml). Precipititation of the salt (e18) could not be induced, so the solvent was evaporated in vacuo, and the residue was chromatographed on a column of silica gel eluting with a gradient of chloroform to 25% ethanol-chloroform. Early fractions contained recovered quaternary ammonium salt (e17) which could be recycled, whilst later fractions contained only the desired sodium salt (e18). Evaporation of the solvents and trituration with ehter afforded the title compound (e18) as a yellow solid (85 mg); $\lambda_{max}$ (EtOH) 364 (9,294) ca. 292 (12,822) and 266 nm (13,847); $\nu_{max}$ (KBr) 1780, 1720 and 1705 $cm^{-1}$; $\delta$(DMF-$d_7$) 1.45 (3H, d, J 6.5 Hz, MeCH), 3.19 (1H, dd, J 10.5 and 19.5 Hz, 4-$CH_a$), 3.83 (1H, dd, J 19.5 and 9Hz, 4-$CH_b$), 3.85 (1H, dd, J 5.5 and 10.5 Hz, 6-CH), 4.45 (1H, m, 5-CH), 4.62 (1H, m, CHMe), 5.46 and 5.62 (each 1H, d, J 13.5Hz, $CH_2Ar$), 7.83 and 8.29 (each 2H, d, J 9Hz, $C_6H_4NO_2$), and 8.84 (1H, s, thiazole-CH).

WHAT WE CLAIM IS:

1. A process for the preparation of a compound of the formula (I):

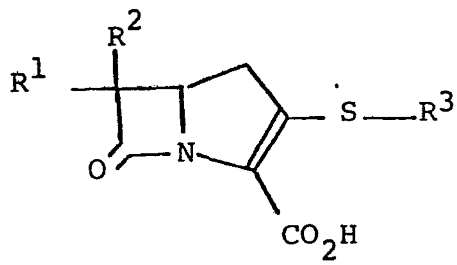

(I)

or a pharmaceutically acceptable salt or in-vivo hydrolysable ester thereof wherein $R^1$ and $R^2$ independently represent a hydrogen atom or an organic group, and $R^3$ represents an optionally substituted aromatic or heteroaromatic moiety; which process comprises the reaction of a compound of the formula (II):

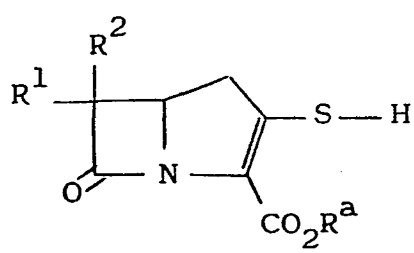

(II)

or reactive derivative thereof, wherein $R^1$ and $R^2$ are as defined in relation to formula (I) and $R^a$ is a carboxy-blocking group or a hydrogen atom; with a compound of the formula (III):

X-$R^3$ (III)

wherein X is a displaceable group, and thereafter if necessary:

i) removing any carboxy-blocking group $R^a$,

ii) converting the product into a pharmaceutically acceptable salt or in-vivo hydrolysable ester.

2. A process as claimed in claim 1 adapted for the preparation of a compound of the formula (I) wherein $R^1$ and $R^2$ are independently selected from hydrogen or substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the linear chain has 1-6 carbon atoms; heteroaralkyl, heterocyclyl and heterocyclylalkyl wherein the substituent or substituents relative to the above-named radicals are selected from the group consisting of: amino, mono-, di- and tri-alkylamino, hydroxyl, alkoxyl, mercapto, alkylthio, arylthio such as phenylthio, sulfamoyl, amidino, guanidino, nitro, chloro, bromo, fluoro, cyano and carboxy, and wherein the hetero atom or atoms in the above-named heterocyclic moieties are selected from the group consisting of 1-4 oxygen, nitrogen or sulphur atoms, and wherein the alkyl moieties of the above-recited substituents have 1-6 carbon atoms.

3. A process as claimed in claim 2 adapted for the preparation of a compound of the formula (I) wherein one of $R^1$ and $R^2$ is a hydrogen atom and the other is sulphonato-oxyethyl or hydroxyalkyl containing up to 6 carbon atoms.

4. A process as claimed in any of claims 1 to 3 adapted for the preparation of a compound of the formula (I) wherein $R^3$ is an optionally substituted ring system selected from phenyl, pyrimidinyl, triazinyl, thiazolyl, pyridyl, thiadiazolyl, oxazolyl, imidazolyl, benzimidazolyl, furyl or benzothiazolyl.

5. A process as claimed in any of claims 1 to 4 wherein $R^3$ is substituted by one or more groups selected from nitro, cyano, chloro, fluoro, iodo, bromo, esterified sulphonyloxy, esterified carboxy, alkanoyl, arylcarbonyl, $C_{1-6}$ alkoxy, aryloxy, aralkoxy, sulphonamido, trifluoromethyl and carbamoyl.

6. A process as claimed in any of claims 1 to 5 wherein X is a chloro, bromo, tosylate or mesylate moiety.

7. A process as claimed in any of claims 1 to 6 wherein the reaction is performed on a thiol of the formula (II) in the presence of anhydrous potassium carbonate.

8. A process as claimed in any of claims 1 to 7 wherein a phase transfer catalyst is present.

9. A process as claimed in claim 1 for the preparation of:
p-Nitrobenzyl (5R,6S)-3-[(4-chloro-6-methyl-pyrimidin-2-yl)-thio]-6-[(S)-1-hydroxyethyl]-7-oxo-1-azabicyclo[3.2.0]-hept-2-ene-2-carboxylate;
p-nitrobenzyl (5R,6S)-3-[4,6-dimethyoxy-1,3,5-triazin-2-ylthio]-6-(S)-1-hydroxyethyl]-7-oxo-1-azabicyclo[3.2.0]-hept-2-ene-2-carboxylate;
p-nitrobenzyl (5R,6R)-3-[4,6-dimethoxy-1,3,5-triazin-2-ylthio]-6-[(S)-1-hydroxyethyl]-7-oxo-1-azabicyclo[3.2.0]-hept-2-ene-2-carboxylate;

(5R,6R)-3-[4,6-dimethoxy-1,3,5-triazin-2-ylthio]-6-[(S)-1-hydroxyethyl]-7-oxo-1-azabicyclo[3.2.0]hept-2-ene-2-carboxylic acid;
p-nitrobenzyl (5R,6R)-3-[5-nitropyrimid-2-yl)thio]-6-[(S)-1-hydroxyethyl]-7-oxo-1-azabicyclo[3.2.0]hept-2-ene-2-carboxylate;
(5R,6R)-3-[(5-aminopyrimid-2-yl)thio]-6-[(S)-1-hydroxyethyl]-7-oxo-1-azabicyclo[3.2.0]hept-2-ene-2-carboxylic acid;
tetra-n-butylammonium salt of p-nitrobenzyl (5R,6R)-6-[(S)-1-hydroxysulphonyloxyethyl]-3-(5-nitropyrimid-2-ylthiol)-7-oxo-1-azabicyclo [3.2.0]hept-2-ene-2-carboxylate;
sodium (5R,6R)-3-(5-aminopyrimidyl-2-thio)-6-[(S)-1-hydroxysulphonyloxyethyl]-7-oxo-1-azabicyclo [3.2.0]hept-2-ene-carboxylate;
p-nitrobenzyl (5R, 6R)-3-[(5-nitrothiazol-2-yl)thio]-6-[(S)-1-hydroxyethyl]-7-oxo-1-azabicyclo[3.2.0]hept-2-ene-2-carboxylate;
p-nitrobenzyl (5R,6R)-3-[(5R,6R)-3-[(5-nitropyrid-2-yl)-thio]-6-[(S)-1-hydroxyethyl]-7-oxo-1-aza-bicyclo[3.2.0]-hept-2-ene-2-carboxylate;
sodium salt of p-nitrobenzyl (5R,6R)-6-[(S)-1-hydroxy-sulphonyloxyethyl]-3-(5-nitrothiazol-2-ylthio)-7-oxo-1-azabicyclo [3.2.0]hept-2-ene-2-carboxylate.

10. A compound of the formula (IV) or a pharmaceutically acceptable salt or _in-vivo_ hydrolysable ester thereof:

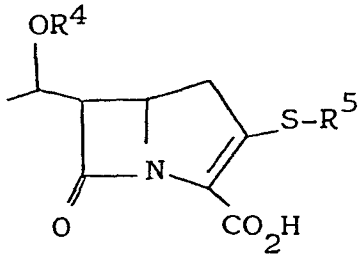

(IV)

wherein $R^4$ is hydrogen or a $SO_3H$ moiety, and $R^5$ is an optionally substituted triazinyl group.

11. A pharmaceutically composition which comprises a compound as claimed in claim 10 and a pharmaceutically acceptable carrier.

European Patent Office

# EUROPEAN SEARCH REPORT

0061231

Application number

EP 82300408.0

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | US - A - 4 232 036 (CHRISTENSEN)<br>* Example 30 *<br>-- | 1 | C 07 D 487/04<br>A 61 K 31/40//<br>(C 07 D 487/04<br>C 07 D 205/00<br>C 07 D 209/00) |
| A | EP - A1 - 0 021 082 (HERCK) (07-01-1981)<br>* Claim 1 *<br>-- | 1 | |
| A | EP - A1 - 0 008 888 (BEECHAM)<br>* Claim 1 *<br>-- | 1 | TECHNICAL FIELDS SEARCHED (Int.Cl. 3) |
| A | HOUBEN-WEYL, MÜLLER "Methoden der organischen Chemie, 4. Auflage, Band IX, 1955<br>GEORG THIEME VERLAG, Stuttgart<br>* Page 102, lines 5-13; Page 103, lines 16-34; Page 117, lines 19-29 *<br>---- | 1 | C 07 D 487/00 |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| VIENNA | 10-05-1982 | HAMMER |